# EUROPÄISCHE PATENTSCHRIFT

(11) **EP 1 593 358 B1**
(45) Veröffentlichungstag und Bekanntmachung des Hinweises auf die Patenterteilung: **25.06.2008**
(21) Anmeldenummer: 05009946.4
(22) Anmeldetag: 06.05.2005
(51) Int. Cl.: A61F 2/60, A61F 2/78

(54) **Rohradapter einer Beinprothese**
Tube adapter for a prosthetic leg
Adaptateur pour tube pour une jambe artificielle

(30) Priorität: 07.05.2004 DE 202004007308 U
(43) Veröffentlichungstag der Anmeldung: 09.11.2005
(73) Patentinhaber: Bauerfeind AG, 07937 Zeulenroda (DE)
(72) Erfinder:
(74) Vertreter: Bardehle, Heinz

(56) Entgegenhaltungen:
- DE-U1- 20 112 189
- DE-U1- 29 505 103
- GB-A- 1 208 421
- US-A- 3 273 168
- "The Electronic C-Leg Knee Joint System" OTTO BOCK , WIEN (AUSTRIA)

## Beschreibung

Die Erfindung bezieht sich auf einen Rohradapter einer Beinprothese mit einem Rohrstück, an dem ein auf das Rohrstück wahlweise weit aufschiebbares Aufnahmeteil für ein dem Kniegelenk zugewandten Prothesenteil festklemmbar ist, und mit einer axial verlaufenden Längenskala.

Ein solcher Rohradapter ist in der DE 295 05 103 U1 beschrieben. Er dient dazu, das den Fuß enthaltende Prothesenteil mit dem dem Kniegelenk zugewandten Prothesenteil zu verbinden. Bei letzterem kann es sich um ein das Kniegelenk ersetzendes Gelenkstück oder einen Teil der Beinprothese handeln, die diese mit dem Kniegelenk verbindet. Durch den Rohradapter lässt sich die Beinprothese hinsichtlich ihrer notwendigen Länge je nach Beinlänge der die Beinprothese tragenden Person und an die Fußstellung dieser Person anpassen, wobei letztere, ausgehend von einer in Gehrichtung weisenden Fußstellung, meist leicht schräg auswärts einstellbar ist. Um diese beiden Einstellungen für den Träger der Beinprothese sichtbar zu machen, so dass er seine Prothese gegebenenfalls nach irgendeiner Behandlung, z.B. Reinigung, wieder neu einstellen kann, hat man bereits bei einem bekannten Rohrstück sowohl eine Längenskala als auch eine Winkelskala aufgetragen, der gegenüber dann die jeweilige Einstellung des Aufnahmeteils abschätzbar ist (siehe Prospekt Otto Bock Health Care "The Electronic C-Leg Knee Joint System" vom August 2001.

Der Erfindung liegt die Aufgabe zugrunde, die Einstellung des Aufnahmeteils zum Rohrstücks, die also für die Länge der eingangs erläuterten Beinprothese und die Winkelstellung des Fußes maßgebend ist, zu vereinfachen. Erfindungsgemäß geschieht dies dadurch, dass das Aufnahmeteil eine umlaufende Winkelskala trägt, wobei die Längslinie der Längenskala mit der Einstellung der Winkelskala durch Verdrehen des Aufnahmeteils in Übereinstimmung bringbar ist.

Bei dieser Gestaltung des Rohradapters einer Beinprothese ist das Aufnahmeteil mit der Winkelskala versehen, so dass über die Einschieblage des Aufnahmeteils und dessen Verdrehwinkel gegenüber dem Rohrstück die gewünschte richtige Einstellung von Aufnahmeteil und Rohrstück zueinander deutlich erkennbar ist. Die Lage des Randes des Rohrstücks in Bezug auf die Längenskala gibt die Länge des Rohradapters an und die Verdrehung der Winkelskala gegenüber der Längslinie der Längenskala zeigt die Stellung des Fußes der Beinprothese an, womit dem Benutzer der Beinprothese deren richtige Einstellung besonders leicht gemacht wird.

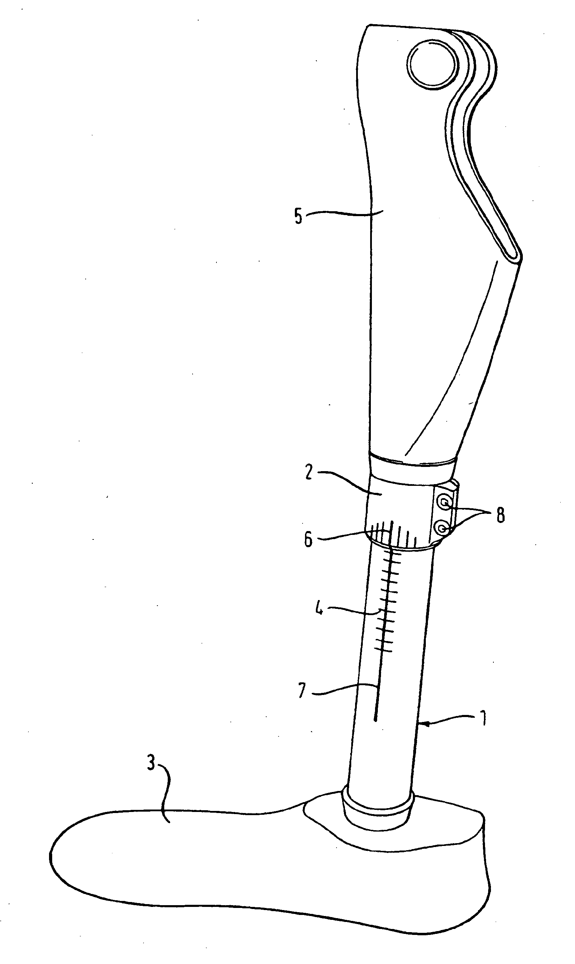

In der Figur ist ein Ausführungsbeispiel der Beinprothese mit dem Rohrstück 1 und dem Aufnahmeteil 2 dargestellt. An das Rohrstück 1 ist an dessen unterem Ende das Fußstück 3 der Beinprothese angesetzt. Auf das dem Fußstück 3 abgewandten Ende des Rohrstücks 1 ist das Aufnahmeteil 2 aufgeschoben, über dessen unteren Rand auf der Längenskala 4 die eingestellte Länge der Beinprothese ablesbar ist. Das Aufnahmeteil 2 trägt an seiner dem Rohrstück 1 abgewandten Seite den Kniegelenkteil 5 der Beinprothese. Am Aufnahmeteil 2 ist die Winkelskala 6 aufgetragen, die in der Darstellung gemäß der Figur mit der Längslinie 7 der Längenskala 4 übereinstimmt, so dass die Beinprothese ihre Normallage bezüglich des Fußstücks 3 einnimmt. Das Aufnahmeteil 2 ist mittels der Klemmschrauben 8 an dem Rohrstück 1 festklemmbar und daher bei Lösen der Klemmschrauben 8 gegenüber dem Rohrstück 1 verdrehbar, wobei sich jeder beliebige Winkel zwischen Aufnahmeteil 2 und Rohrstück 1 einstellen lässt, womit die erforderliche Anpassung der Beinprothese an die Fußrichtung des Trägers der Beinprothese mit Hilfe der Längslinie 7 gegenüber der Winkelskala 6 ablesbar ist.

## Patentansprüche

1. Rohradapter einer Beinprothese mit einem Rohrstück (1), an dem ein auf das Rohrstück (1) wahlweise weit aufschiebbares Aufnahmeteil (2) für ein dem Kniegelenk (5) zugewandten Prothesenteil festklemmbar ist, und mit einer axial verlaufenden Längenskala (4), **dadurch gekennzeichnet, dass** das Aufnahmeteil (2) eine umlaufende Winkelskala (6) trägt, wobei eine Längslinie (7) an der am Rohrstück (1) angebrachten Längenskala (4) mit der Einteilung der Winkelskala (6) durch Verdrehen des Aufnahmeteils (2) in Übereinstimmung bringbar ist.

## Claims

1. Tube adapter for a prosthetic leg, comprising a tube section (1) to which a receiving part (2) which is intended for a part of the prosthesis directed towards the knee joint (5) and which can be slipped as far as required on to the tube section (1) can be clamped, and comprising an axially extending length scale (4), **characterized in that** the receiving part (2) has a circumferential angle scale (6), wherein a vertical line (7) on the length scale (4) provided on the tube section (1) can be aligned with the graduation of the angle scale (6) by rotating the receiving part (2).

## Revendications

1. Adaptateur tubulaire d'une prothèse de jambe comportant une pièce tubulaire (1), auquel peut être assujettie une partie réceptrice (2), coulissant à volonté en éloignement sur la partie tubulaire (1), pour une partie de prothèse tournée vers l'articulation du genou, et comportant une échelle de longueur (4) s'étendant axialement (4), **caractérisé en ce que** la partie réceptrice (2) porte un échelle angulaire circonférentielle (6), un trait longitudinal (7) sur l'échelle de longueur (4) formée sur la partie tubulaire (1) pouvant être amené en coïnciderice avec les graduations de l'échelle angulaire (6) par rotation de la partie réceptrice (2).
